(19) **Europäisches Patentamt / European Patent Office / Office européen des brevets**

(11) **EP 4 759 791 A1**

(12) ## EUROPEAN PATENT APPLICATION
published in accordance with Art. 153(4) EPC

(43) Date of publication:
**17.06.2026  Bulletin 2026/25**

(21) Application number: **24851940.7**

(22) Date of filing: **08.08.2024**

(51) International Patent Classification (IPC):
*C07C 4/22* (2006.01)  *B01J 29/18* (2006.01)
*B01J 29/70* (2006.01)  *B01J 37/02* (2006.01)
*B01J 37/08* (2006.01)  *C07B 61/00* (2006.01)
*C07C 11/02* (2006.01)  *C08J 11/10* (2006.01)
*C10G 1/10* (2006.01)

(52) Cooperative Patent Classification (CPC):
**B01J 29/18; B01J 29/70; B01J 37/02; B01J 37/08; C07B 61/00; C07C 4/22; C07C 11/02; C08J 11/10; C10G 1/10;** Y02W 30/62

(86) International application number:
**PCT/JP2024/028594**

(87) International publication number:
**WO 2025/033523 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:  **10.08.2023  JP 2023131008**

(71) Applicant: **Resonac Corporation
Tokyo 105-7325 (JP)**

(72) Inventors:
• **UEDA, Yasuyuki
Tokyo 105-7325 (JP)**

• **SATO, Takashi
Tokyo 105-7325 (JP)**
• **TEZUKA, Noriyasu
Tokyo 105-7325 (JP)**
• **TAKASHI, Hiroko
Tokyo 105-7325 (JP)**
• **MINAMI, Takuya
Tokyo 105-7325 (JP)**

(74) Representative: **Hoffmann Eitle
Patent- und Rechtsanwälte PartmbB
Arabellastraße 30
81925 München (DE)**

(54) **METHOD FOR PRODUCING OLEFIN-CONTAINING COMPOSITION AND CATALYST FOR PRODUCTION OF OLEFIN-CONTAINING COMPOSITION**

(57)  A method for producing an olefin-containing composition includes a step of decomposing plastic in the presence of a zeolite to produce an olefin-containing composition including an olefin with 2 to 5 carbon atoms, wherein the zeolite is at least one selected from the group consisting of a beta zeolite including $Rb^+$ and a mordenite zeolite including $Rb^+$.

## Description

TECHNICAL FIELD

The present invention relates to methods for producing olefin-containing compositions and catalysts for producing olefin-containing compositions.

BACKGROUND ART

Plastic waste has been processed through landfill, ocean dumping, incineration, or the like. However, it is getting more difficult to secure landfill sites, and ocean dumping causes environmental problems as plastic is not decomposed. Further, although heat from incineration of plastic can be utilized, incineration has a problem associated with emission of carbon dioxide gas, which leads to global warming.

Therefore, recycling of plastic waste, such as reuse, regeneration, and the like, has been desired in recent years from rising awareness in environmental issues. Research and development on plastic waste recycling have been actively carried out. In addition, most plastic materials are produced by fossil fuels, and establishment of a recycling method is also strongly desired in view of effective use of resources.

Among plastic materials, hydrocarbon-based plastic materials, such as polyethylene (PE), polypropylene (PP), polystyrene (PS), and the like are widely used for drink and food containers, packaging materials, molded article, films, and the like because of excellent properties, such as heat resistance, weather resistance, mechanical strength, transparency, chemical resistance, gas barrier properties, and the like. Therefore, the hydrocarbon-based plastic materials occupy the majority of the plastic materials constituting plastic material waste. As a recycling method for these plastic materials, there is chemical recycling that uses pyrolysis or gasification. In the pyrolysis or gasification, the plastic waste is turned into low molecular hydrocarbons by thermal decomposition, and many methods using a solid catalyst for improving reactivity or selectivity of resultant products have been studied.

For example, it is disclosed that, in a method for inductively heating a plastic material, a catalyst, and a magnetic susceptor using a radio frequency (RF) field, a nickel-doped beta zeolite (Ni-BEA) used as the catalyst achieves a lower reaction speed than a hydrogen-containing beta zeolite (H-BEA) (see Patent Document 1). In addition, it is disclosed that, in a thermal decomposition method of tires, ethylene and propylene yields are higher with a 1% palladium-loaded beta zeolite (1%PD/HBeta), and are yet higher with a 10% nickel-loaded beta zeolite (10%Ni/HBeta), compared to a case of no catalyst or a beta zeolite loading no metal. Further, it is disclosed that yields of single-ring aromatics in a liquid phase are higher with 1%PD/HBeta, and are also slightly higher with 5%Ni/HBeta and 10%Ni/HBeta compared with the beta zeolite loading no metal (see Non-Patent Document 1).

RELATED ART DOCUMENTS

PATENT DOCUMENT

Patent Document 1: International Publication No. WO 2023/064741

NON-PATENT DOCUMENT

Non-Patent Document 1: Z Wang et al., International Journal of Metallurgical and Materials Engineering, 2021, Vol. 6, No. 4, pp. 260-264

DISCLOSURE OF THE INVENTION

PROBLEMS TO BE SOLVED BY THE INVENTION

However, even when the catalysts disclosed in the related art documents are used, a sufficiently satisfactory yield of useful components, such as lower olefins, has not been achieved.

The present disclosure aims to provide a method for producing an olefin-containing composition with a high yield of useful components.

MEANS FOR SOLVING THE PROBLEMS

A method for producing an olefin-containing composition according to one aspect of the present disclosure includes a step of decomposing plastic in the presence of a zeolite to produce an olefin-containing composition including an olefin with 2 to 5 carbon atoms, wherein the zeolite is at least one selected from the group consisting of a beta zeolite including $Rb^+$ and a mordenite zeolite including $Rb^+$.

The means for solving the above problems are as follows.

<1> A method for producing an olefin-containing composition, the method including:
a step of decomposing plastic in the presence of a zeolite to produce an olefin-containing composition including an olefin with 2 to 5 carbon atoms,
wherein the zeolite is at least one selected from the group consisting of a beta zeolite including $Rb^+$ and a mordenite zeolite including $Rb^+$.

<2> The method for producing the olefin-containing composition according to <1>,
wherein the plastic is polyolefin.

<3> The method for producing the olefin-containing composition according to <1>,
wherein the plastic includes at least one selected from the group consisting of polyethylene, polypropylene, and polystyrene.

<4> The method for producing the olefin-containing composition according to any one of <1> to <3>,
wherein the decomposing of the plastic is performed at a temperature of 300°C or higher and 1,100°C or lower.

<5> The method for producing the olefin-containing composition according to any one of <1> to <4>,
wherein a molar ratio [$SiO_2/Al_2O_3$] of $SiO_2$ to $Al_2O_3$ of each of the beta zeolite including $Rb^+$ and the mordenite zeolite including $Rb^+$ is 10 or greater and 10,000 or less.

<6> The method for producing the olefin-containing composition according to any one of <1> to <5>,

wherein the beta zeolite including $Rb^+$ has a BET specific surface area of 400 $m^2/g$ or greater and 500 $m^2/g$ or less, and
the mordenite zeolite including $Rb^+$ has a BET specific surface area of 300 $m^2/g$ or greater and 400 $m^2/g$ or less.

<7> The method for producing the olefin-containing composition according to any one of <1> to <6>, further including:

a mixing step of obtaining a liquid mixture in which at least one selected from the group consisting of a beta zeolite and a mordenite zeolite, and a liquid including a rubidium compound are mixed; and
a calcination step of calcining the liquid mixture in the air to obtain at least one selected from the group consisting of the beta zeolite including $Rb^+$ and the mordenite zeolite including $Rb^+$.

<8> The method for producing the olefin-containing composition according to <7>,
wherein the mixing step satisfies $1 \le yz/x \le 5,000$, where x (g) is a mass of at least one selected from the group consisting of the beta zeolite including $Rb^+$ and the mordenite zeolite including $Rb^+$, y is a molar ratio [$SiO_2/Al_2O_3$] of $SiO_2$ to $Al_2O_3$ of at least one selected from the group consisting of the beta zeolite including $Rb^+$ and the mordenite zeolite including $Rb^+$, and z (mmol) is a number of moles of the rubidium compound.

<9> The method for producing the olefin-containing composition according to <7> or <8>,

wherein the beta zeolite is a beta zeolite including $H^+$, and
the mordenite zeolite is a mordenite zeolite including $H^+$.

<10> A catalyst for producing an olefin-containing composition, including:
at least one selected from the group consisting of a beta zeolite including $Rb^+$ and a mordenite zeolite including $Rb^+$,
wherein the catalyst is used for producing an olefin-containing composition that includes an olefin with 2 to 5 carbon atoms.

<11> A method of using a catalyst, the method including:
using the catalyst that includes at least one selected from the group consisting of a beta zeolite including $Rb^+$ and a mordenite zeolite including $Rb^+$ in production of an olefin-containing composition that includes an olefin with 2 to 5 carbon atoms.

EFFECTS OF THE INVENTION

According to embodiments of the present disclosure, a method for producing an olefin-containing composition with a high yield of useful components can be provided.

MODE OF CARRYING OUT THE INVENTION

The present disclosure will be described in detail hereinafter. Embodiments of the present disclosure are not limited by the following description, and can be appropriately modified without departing from the gist of the present disclosure. In the present specification, "to", which indicates a numerical range, encompasses that numerical values described before and after "to" are included as a lower limit and an upper limit.

(Method for producing olefin-containing composition)

The method for producing the olefin-containing composition according to one embodiment includes a step of decomposing plastic in the presence of a zeolite to produce an olefin-containing composition including an olefin with 2 to 5 carbon atoms (may be referred to as a "decomposition step" hereinafter), and may further include other steps as necessary.

<Decomposition step>

The decomposition step is a step of decomposing plastic in the presence of a zeolite to produce an olefin-containing composition including an olefin with 2 to 5 carbon atoms.
The zeolite includes at least one selected from the group consisting of a beta zeolite including $Rb^+$ and a mordenite zeolite including $Rb^+$. Thus, an olefin-containing composition, which achieves an excellent yield of useful components, such as olefins with 2 to 5 carbon atoms, benzene, toluene, ethyl benzene, xylene, styrene, cumene, and the like, compared to a known beta or mordenite zeolite including $Rb^+$ in the related art, can be produced.
The usage amount of the zeolite relative to the plastic in the decomposition step is not particularly limited, and may be appropriately selected according to the intended purpose. The usage amount of the zeolite is preferably 100 parts by mass or less, more preferably 1 part by mass or greater and 100 parts by mass or less, yet more preferably 10 parts by mass or greater and 100 parts by mass or less, yet even more preferably 20 parts by mass or greater and 100 parts by mass or less, and particularly preferably 1 part by mass or greater and 30 parts by mass or less, relative to 100 parts by mass of the plastic. When the usage amount of the zeolite relative to the plastic is 10 parts by mass or greater and 100 parts by mass or less relative to 100 parts by mass of the plastic, the plastic can be suitably decomposed, and a favorable useful component yield is achieved.

<<Olefin-containing composition>>

The olefin-containing composition includes an olefin with 2 to 5 carbon atoms, preferably further includes an aromatic compound, and may further include other components, as necessary.

-Olefin with 2 to 5 carbon atoms-

The olefin with 2 to 5 carbon atoms preferably includes, as a main component, an olefin including at least one selected from the group consisting of alkenes and dienes, more preferably at least one selected from the group consisting of an alkene with 2 to 5 carbon atoms and a diene with 3 to 5 carbon atoms, and yet more preferably an alkene with 2 to 5 carbon atoms.
Examples of the olefin with 2 carbon atoms include ethylene.
Examples of the olefin with 3 carbon atoms include propylene.
Examples of the olefin with 4 carbon atoms include 1-butene, cis-2-butene, trans-2-butene, and 2-methylpropene.
Examples of the olefin with 5 carbon atoms include 1-pentene, cis-2-pentene, trans-2-pentene, 2-methyl-1-butene, and 2-methyl-2-butene.
In the present disclosure, the olefin with 2 to 5 carbon atoms may be referred to as a "lower olefin".
The lower olefin serves as a raw material for polyolefin. The polyolefin can be suitably used as a raw material in various fields, such as plastic bags, plastic wrapping films, straws, medical devices, home appliance housings, erasers, hosepipes, tires, tubes, CD cases, food trays, food containers, PET bottles, fibers, and the like.
An amount of the lower olefins in the olefin-containing composition is not particularly limited, and may be appropriately selected according to the intended purpose. The amount of the lower olefins is preferably 30 percent by mass or greater and 100 percent by mass or less, and more preferably 35 percent by mass or greater and 60 percent by mass or less.

-Aromatic compound-

The aromatic compound is not particularly limited, and may be appropriately selected according to the intended purpose. The aromatic compound preferably includes at least one selected from the group consisting of benzene, toluene, ethylbenzene, three positional isomers of xylene (p-xylene, m-xylene, and o-xylene), styrene, and cumene.

In the present disclosure, the aromatic compound may be referred to as "useful aromatics".

The useful aromatics serve as a raw material for resins, and the resins can be suitably used as a raw material in various fields, such as CD cases, food trays, food containers, PET bottles, fibers, and the like.

The amount of the useful aromatics in the olefin-containing composition is not particularly limited, and may be appropriately selected according to the intended purpose. The amount of the useful aromatics is preferably 0 percent by mass or greater and 50 percent by mass or less, more preferably 0.1 percent by mass or greater and 30 percent by mass or less, and yet more preferably 1 percent by mass or greater and 20 percent by mass or less.

In the present disclosure, the lower olefins and the useful aromatics may be collectively referred to as "useful components".

--Useful component yield--

In the present disclosure, the useful component yield is determined by analyzing a tetrahydrofuran (may be abbreviated as "THF" hereinafter)-soluble product (may be referred to as a "THF-soluble product" hereinafter) from the olefin-containing composition obtained in the decomposition step, specifically, a gaseous product and residue, under the following analysis conditions using a gas chromatography (may be abbreviated as "GC" hereinafter) device equipped with a flame ionization detector, and quantifying each component based on a ratio between a peak area of each component and a peak area of an internal standard substance according to an internal standard method. The internal standard substance is not particularly limited, as long as a substance for use is stable under the analysis conditions, and is easily separated from a substance to be analyzed. For example, at least one selected from the group consisting of cyclopentane and t-butyl benzene can be used as the internal standard substance. The GC analysis of the gaseous product and THF-soluble product is performed under the analysis conditions described in Examples.

In the case where the decomposition step is performed in a sealed reaction vessel, the THF-soluble product is obtained by adding THF to the residues in the reaction vessel. In the case where the decomposition step is performed in a continuous or open reaction vessel, the gaseous product and the residues can be recovered not only from the inside of the reaction vessel, but also from the trap outside the reaction vessel, and therefore the THF-soluble product is obtained by adding THF to the residues inside the reaction vessel, and adding THF to the trap and the connecting member between the reaction vessel and the trap.

The useful component yield is preferably 35% or greater, more preferably 40% or greater.

The useful component yield is determined as a ratio of the number of moles of the carbon atoms in the useful components to the number of moles of the carbon atoms in the plastic according to the following equation.

Useful component yield (%) = a mass [g] of the generated useful components calculated by GC analysis/a total mass [g] of a plastic sample $\times$ 100

--O/P ratio--

In the present disclosure, in addition to a high useful component yield, the amount of paraffins as a by-product is preferably small. The fact that the amount of paraffins as a by-product is small and the yield of olefins with 2 to 5 carbon atoms is excellent can be evaluated by determining a ratio of a total yield (%) of an olefin product with 2 to 5 carbon atoms to a total yield (%) of a paraffin product with 2 to 5 carbon atoms, i.e., a ratio [the total yield (%) of the olefin product with 2 to 5 carbon atoms/the total yield (%) of the paraffin product with 2 to 5 carbon atoms] (may be referred to as an "O/P ratio" hereinafter). In the present disclosure, the term "paraffin" refers to a saturated aliphatic hydrocarbon with 2 to 5 carbon atoms, preferably a saturated chain aliphatic hydrocarbon with 2 to 5 carbon atoms.

The O/P ratio is not particularly limited, and may be appropriately selected according to the intended purpose, but is preferably 0.75 or greater, and more preferably 0.8 or greater. A higher numerical value for the O/P ratio is more preferable, and therefore the upper limit is not particularly limited. The upper limit is preferably 50 or less, and more preferably 20 or less.

The O/P ratio can be determined from the amount of each component obtained in the measurement of the useful component yield.

-Other components-

Examples of the other components in the olefin-containing composition include decomposition products of the plastic other than the zeolite and the useful components, undecomposed plastic, and materials or additives included in the raw materials other than the plastic.

The other components in the olefin-containing composition may be appropriately removed by a method known in the related art when the useful components are recycled.

<<Zeolite>>

The framework structures of the zeolite are stored in a database by International Zeolite Association (may be abbreviated as "IZA" hereinafter), and the IUPAC structure codes (may be abbreviated as "structure codes" hereinafter) of the framework structures are defined. According to the structure codes, the beta zeolite is represented by "BEA" and the mordenite zeolite is represented by "MOR".

The crystal systems of the BEA zeolite and the MOR zeolite can be determined by analyzing the zeolite by X-ray diffraction (XRD) under the analysis conditions described in Examples. In addition, the crystal systems can also be determined by comparison to XRD patterns disclosed in Collection of simulated XRD powder patterns for zeolites, Fifth revised edition (2007) or XRD patterns disclosed in Zeolite Framework Types on the website of the IZA Structure Commission (http://www.iza-struture.org/databases/) .

-Pore size-

The pore size of $Al_2O_3$ of the BEA zeolite including $Rb^+$ is generally approximately 0.65 nm.

The pore size of $Al_2O_3$ of the MOR zeolite including $Rb^+$ is generally approximately 0.7 nm.

The pore size of each of the BEA zeolite including $Rb^+$ and the MOR zeolite including $Rb^+$ can be measured according to ISO 15901-3:2007 "Pore size distribution and porosity of solid materials by mercury porosimetry and gas adsorption-Part 3: Analysis of micropores by gas adsorption)".

-Molar ratio $[SiO_2/Al_2O_3]$-

The molar ratio $[SiO_2/Al_2O_3]$ of the number of moles of $SiO_2$ to the number of moles of $Al_2O_3$ of the BEA zeolite including $Rb^+$ is not particularly limited, and may be appropriately selected according to the intended purpose, but is preferably 10 or greater and 10,000 or less, more preferably 20 or greater and 10,000 or less, yet more preferably 20 or greater and 5,000 or less, and particularly preferably 500 or greater and 2,000 or less. When the molar ratio $[SiO_2/Al_2O_3]$ of the BEA zeolite including $Rb^+$ is 10 or greater and 10,000 or less, a favorable useful component yield is achieved.

The molar ratio $[SiO_2/Al_2O_3]$ of the number of moles of $SiO_2$ to the number of moles of $Al_2O_3$ of the MOR zeolite including $Rb^+$ is not particularly limited and may be appropriately selected according to the intended purpose, but is preferably 10 or greater and 10,000 or less, more preferably 10 or greater and 10,000 or less, yet more preferably 10 or greater and 5,000 or less, and particularly preferably 10 or greater and 2,000 or less. When the molar ratio $[SiO_2/Al_2O_3]$ of the MOR zeolite including $Rb^+$ is 10 or greater and 10,000 or less, a favorable useful component yield is achieved.

The molar ratio $[SiO_2/Al_2O_3]$ can be calculated, for example, by accurately weighing the BEA zeolite including $Rb^+$ or the MOR zeolite including $Rb^+$, completely dissolving the BEA zeolite including $Rb^+$ or the MOR zeolite including $Rb^+$ in an aqueous solution including nitric acid and hydrofluoric acid, measuring a volume of a resultant sample, measuring amounts of Si and Al by ICP optical emission spectrometer (e.g., PlasmaQuant PQ 9000, manufactured by Analytik Jena AG), and calculating a molar ratio from the number of moles of Si and Al calculated from the amounts of Si and Al according to the following equation.

Molar ratio $[SiO_2/Al_2O_3] = 2 \times$ (number of moles of Si)/(number of moles of Al)

-BET specific surface area-

The BET specific surface area of the BEA zeolite including $Rb^+$ is not particularly limited and may be appropriately selected according to the intended purpose, but is preferably 100 $m^2/g$ or greater and 1,000 $m^2/g$ or less, more preferably 100 $m^2/g$ or greater and 600 $m^2/g$ or less, yet more preferably 200 $m^2/g$ or greater and 500 $m^2/g$ or less, and particularly preferably 300 $m^2/g$ or greater and 500 $m^2/g$ or less. When the BET specific surface area of the BEA zeolite including $Rb^+$ is 100 $m^2/g$ or greater and 1,000 $m^2/g$ or less, a favorable useful component yield is achieved.

The BET specific surface area of the MOR zeolite including $Rb^+$ is not particularly limited and may be appropriately selected according to the intended purpose, but is preferably 100 $m^2/g$ or greater and 1,000 $m^2/g$ or less,

more preferably 100 m²/g or greater and 600 m²/g or less, yet more preferably 200 m²/g or greater and 500 m²/g or less, and particularly preferably 300 m²/g or greater and 400 m²/g or less. When the BET specific surface area of the MOR zeolite including Rb⁺ is 100 m²/g or greater and 1,000 m²/g or less, a favorable useful component yield is achieved.

The BET specific surface area of the BEA zeolite including Rb⁺ and the BET specific surface area of the MOR zeolite including Rb⁺ can be measured according to ISO 9277:2010 "Determination of the specific surface area of powder (solids) by gas adsorption".

-Particle size-

In the case where a powdery BEA zeolite including Rb⁺ is used as the BEA zeolite including Rb⁺, a 50% particle size D50 (median diameter) (volume average) of the BEA zeolite containing Rb⁺ is not particularly limited, and may be appropriately selected according to the intended purpose. From the viewpoint of the BET specific surface area, handling, and the like, the 50% particle size D50 is preferably 1 μm or greater and 500 μm or less, more preferably 2 μm or greater and 350 μm or less, and yet more preferably 2 μm or greater and 100 μm or less.

In the case where a powdery MOR zeolite including Rb⁺ is used as the MOR zeolite including Rb⁺, a 50% particle size D50 (median diameter) (volume average) of the MOR zeolite containing Rb⁺ is not particularly limited, and may be appropriately selected according to the intended purpose. From the viewpoint of the BET specific surface area, handling, and the like, the 50% particle size D50 is preferably 1 μm or greater and 500 μm or less, more preferably 2 μm or greater and 350 μm or less, and yet more preferably 2 μm or greater and 100 μm or less.

In the present disclosure, the term "powdery" encompasses at least one selected from the group consisting of primary particles, aggregates of primary particles (secondary particles), granules obtained by granulating primary particles, and granules obtained by granulating secondary particles.

The 50% particle size D50 of each of the BEA zeolite including Rb⁺ and the MOR zeolite including Rb⁺ refers to a median diameter measured by a laser diffraction particle size analyzer (e.g., a laser diffraction particle size analyzer SALD-7100, manufactured by Shimadzu Corporation).

The particle shape of each of the BEA zeolite including Rb⁺ and the MOR zeolite including Rb⁺ is not particularly limited. Examples of the particle shape include a spherical shape, an ellipsoidal shape, a crushed shape, a flat shape, an irregular shape, and the like. One of the above shapes may be used, or two or more of the above shapes may be used in combination.

-Method of producing BEA zeolite including Rb⁺ or MOR zeolite including Rb⁺-

The method of producing the BEA zeolite including Rb⁺ or the MOR zeolite including Rb⁺ is not particularly limited, and may be appropriately selected from methods known in the related technical field. Rb⁺ can be introduced by processing a BEA or MOR zeolite including no Rb⁺ with an Rb⁺ source according to the below-described method of the present disclosure. In another embodiment, for example, a BEA or MOR zeolite can be produced by adding Rb⁺ in advance.

--Method of producing BEA or MOR zeolite--

Examples of a method of producing a typical BEA or MOR zeolite include a method in which a hydrothermal synthesis is performed using a mixture (raw material composition) including a silica source, an alumina source, an organic structure-directing agent, a hydroxide-containing compound, a fluoride ion-containing compound, water, and the like, and an Rb source in the case where Rb⁺ is added in advance. After the hydrothermal synthesis, solid-liquid separation, water washing, drying in which moisture is removed, for example, in the air of approximately 50°C to 150°C, and the like may be further performed according to common practice, as necessary. In addition, the organic structure-directing agent may be removed by firing that is performed to the extent that the framework of the zeolite is not collapsed. The firing temperature in this case is preferably 500°C to 600°C.

The silica source is not particularly limited, but is preferably precipitated silica, colloidal silica, fumed silica, silica gel, sodium silicate (e.g., sodium metasilicate, sodium orthosilicate, sodium silicate No. 1, sodium silicate No. 2, sodium silicate No. 3, sodium silicate No. 4, or the like), alkoxysilane (e.g., tetraethoxysilane (TEOS), trimethylethoxysilane (TMEOS), or the like), or the like, more preferably tetraethoxysilane (TEOS) or trimethylethoxysilane (TMEOS), and yet more preferably tetraethoxysilane (TEOS). The above silica sources may be used alone or in combination.

The alumina source is not particularly limited, but is preferably aluminum chloride, aluminum nitrate, aluminum sulfate, sodium aluminate, aluminum alkoxide (e.g., aluminum isopropoxide or the like), or aluminum hydroxide (e.g., boehmite or the like), and more preferably aluminum nitrate. The above alumina sources may be used alone or in combination.

The organic structure-directing agent is not particularly limited. For example, in the case where a BEA zeolite is produced, a tetraethylammonium compound is used. In the case where a MOR zeolite is produced, at least one selected from the group consisting of a tetraethyl ammonium compound and a benzyl trimethylammonium compound is used.

The hydroxide-containing compound or the fluoride ion-containing compound is added for the purpose of facilitating crystallization of the zeolite. The hydroxide-containing compound is widely used because the hydroxide-containing compound is inexpensive and easily handled. The hydroxide-containing compound is not particularly limited, as long as the hydroxide-containing compound is alkaline in an aqueous solution.

For example, the organic structure-directing agent can also serve as the hydroxide-containing compound. In this case, a hydroxide of the above-described tetrapropylammonium compound or tetraethyl ammonium compound is used. According to the above method, an alkali metal is not introduced into the zeolite, thereby producing a protonic zeolite.

However, an alkali metal ion can be introduced into a zeolite. In the case where it is desired to actively introduce alkali metal ions into a zeolite, in addition to the organic structure-directing agent, a hydroxide of an alkali metal or an alkaline earth metal, preferably a hydroxide of an alkali metal, is added as the hydroxide-containing compound. Examples of the organic structure-directing agent used in this case include tetrapropylammonium bromide and tetraethylammonium bromide. Examples of the hydroxide of the alkali metal include lithium hydroxide, sodium hydroxide, potassium hydroxide, rubidium hydroxide, and cesium hydroxide.

An $Rb^+$ source used when a BEA zeolite or MOR zeolite is produced by adding $Rb^+$ in advance is not particularly limited, and examples of the $Rb^+$ source include a rubidium compound. Specifically, rubidium hydroxide may be used as the hydroxide, which can also serve as the $Rb^+$ source. As a method of introducing alkali metal ions, such as $Rb^+$, an alkali metal compound other than an oxide may be added. In this case, the hydroxide or fluoride ion-containing compound is additionally added to facilitate crystallization of the zeolite. In the case where alkali metal ions are added using the $Rb^+$ source or another alkali metal compound during production of the zeolite, the alkali metal compound is likely to be excessive, and therefore washing is performed with water after the hydrothermal synthesis, thereby washing off and removing the excessive alkali metal ions. The above $Rb^+$ source is preferably added before the hydrothermal synthesis.

Water used in the hydrothermal synthesis is not particularly limited. Examples of the water include industrial water, tap water, distilled water, deionized water, pure water, RO water (reverse osmosis membrane treated water), and ultrapure water. The above examples of the water may be used alone or in combination.

The hydrothermal synthesis is generally performed in a reaction vessel. As the reaction vessel used in the hydrothermal synthesis, any known reaction vessel known in the related art can be used as long as it is a sealed pressure-resistant vessel used for a hydrothermal synthesis, and a type of the reaction vessel is not particularly limited. For example, a sealed heat-resistant and pressure-resistant vessel, such as an autoclave equipped with a stirring device, a heat source, a pressure gauge, and a safety valve, or the like is preferably used. The crystallization of the zeolite may be performed in a state in which the mixture is left to stand. From the viewpoint of improving uniformity of a resultant zeolite, the crystallization is preferably performed in a state in which the mixture is stirred and mixed.

A processing temperature (reaction temperature) of the hydrothermal synthesis is not particularly limited, and may be appropriately selected from the viewpoint of crystallinity of a resultant zeolite, cost efficiency, and the like. The processing temperature is preferably 100°C or higher and 200°C or lower, more preferably 120°C or higher and 190°C or lower, and yet more preferably 150°C or higher and 180°C or lower.

A processing time (reaction time) of the hydrothermal synthesis is not particularly limited as long as crystallization can be performed, and may be appropriately selected from the viewpoint of crystallinity of a resultant zeolite, cost efficiency, and the like. The processing time is preferably 1 hour or longer and 20 days or shorter, more preferably 4 hours or longer and 15 days or shorter, and yet more preferably 12 hours or longer and 11 days or shorter.

A processing pressure of the hydrothermal synthesis is not particularly limited. The autogenous pressure, which is generated when the mixture in the reaction vessel is heated in the above temperature range, is sufficient to be used as the processing pressure. At this time, an inert gas, such as a nitrogen gas, an argon gas, or the like, may be introduced into the vessel as necessary.

The zeolite may include non-metal cations, such as ammonium ions ($NH_4^+$), protons ($H^+$), and the like. A method of performing ion exchange between the non-metal cations and $Rb^+$ of the $Rb^+$ source is not particularly limited, and can be performed according to common practice.

--Method of producing BEA zeolite including Rb+ using BEA zeolite as raw material and method of producing MOR zeolite including Rb+ using MOR zeolite as raw material--

Each of the method of producing the BEA zeolite including $Rb^+$ and the method of producing the MOR zeolite including $Rb^+$ of the present disclosure includes a mixing step and a calcination step, preferably further includes a stirring step and an evaporation drying step, and further includes other steps as necessary.

---Mixing step---

The mixing step is a step of obtaining a liquid mixture in which at least one selected from a BEA zeolite and a MOR zeolite (may be referred to as a "raw material zeolite" hereinafter) and a liquid including a rubidium compound are mixed.

By performing the mixing step, for example, a cation ($Rb^+$) included in the rubidium compound can be bonded to an acid site of the raw material zeolite via ionic bonding.

The rubidium compound is not particularly limited. Examples of the rubidium compound include rubidium hydroxide, rubidium nitrate, rubidium chloride, and rubidium carbonate. The above rubidium compounds may be used alone or in combination. Among the above rubidium compounds, the rubidium compound is preferably rubidium nitride because of excellent solubility.

The raw material zeolite used in the mixing step may be an acidic zeolite or a basic zeolite. Between the acidic zeolite and the basic zeolite, an acidic zeolite including Bronsted acid sites is preferably used as the raw material zeolite because ion exchange with cations efficiently progresses when the liquid including the rubidium compound is used.

The raw material zeolite used in the mixing step is not particularly limited, and may be appropriately synthesized or a commercial product.

The cations included in the raw material zeolite are not particularly limited. However, at least one selected from the group consisting of a BEA zeolite including $H^+$ and a MOR zeolite including $H^+$ is preferable in view of favorable substitution efficiency with $Rb^+$.

In the case where a BEA zeolite including $H^+$ is used as a raw material of the BEA zeolite including $Rb^+$, a quantity of acid sites of the BEA zeolite including $H^+$ is not particularly limited, and may be appropriately selected according to the intended purpose. The quantity of the acid sites of the BEA zeolite including $H^+$ is preferably 0.05 mmol/g or greater and 2.5 mmol/g or less, more preferably 0.05 mmol/g or greater and 2.0 mmol/g or less, and yet more preferably 0.05 mmol/g or greater and 0.1 mmol/g or less. When the quantity of acid sites of the BEA zeolite including $H^+$ is 0.05 mmol/g or greater and 2.5 mmol/g or less, a BEA zeolite including $Rb^+$ achieving a favorable useful component yield is obtained.

In the case where a MOR zeolite including $H^+$ is used as a raw material of the MOR zeolite including $Rb^+$, a quantity of acid sites of the BEA zeolite including $H^+$ is not particularly limited, and may be appropriately selected according to the intended purpose. The quantity of the acid sites of the MOR zeolite including $H^+$ is preferably 0.5 mmol/g or greater and 2.5 mmol/g or less, more preferably 1.0 mmol/g or greater and 2.5 mmol/g or less, and yet more preferably 1.0 mmol/g or greater and 2.0 mmol/g or less. When the quantity of acid sites of the MOR zeolite including $H^+$ is 0.5 mmol/g or greater and 2.5 mmol/g or less, a MOR zeolite including $Rb^+$ achieving a favorable useful component yield is obtained.

The quantity of acid sites of each of the BEA zeolite including $H^+$ and the MOR zeolite including $H^+$ can be measured by ammonia temperature programmed deposition (may be abbreviated as "TPD" hereinafter).

A method of synthesizing the raw material zeolite is not particularly limited, and may be appropriately selected from methods known in the related art. Examples of the method include a method in which an aluminum nitrate aqueous solution is added to tetraalkoxysilane to carry out hydrolysis, and after removing generated alcohol, a hydrothermal synthesis is performed, followed by performing calcination.

A solvent used in the liquid including the rubidium compound is not particularly limited, but is preferably a solvent that can dissolve the rubidium compound, can be relatively easily removed in a below-described calcination step, and is not decomposed, and more preferably a highly polar solvent. Examples of such a solvent include water, methanol, ethanol, acetonitrile, dimethylformamide, dimethylacetamide, dimethylsulfoxide, and hexamethylphosphoramide. The above solvents may be used alone or in combination. Among the above solvents, at least one selected from the group consisting of water, methanol, and ethanol is preferable in view of a relatively low boiling point and easy removal, and water is particularly preferable in view of low cost.

The concentration of the rubidium compound in the liquid including the rubidium compound is not particularly limited, and may be appropriately selected according to the intended purpose. In view of reduction in cost of the solvent, the concentration is preferably 0.01 mol/L or greater and 10 mol/L or less, more preferably 0.05 mol/L or greater and 2.5 mol/L or less. When the concentration of the rubidium compound is 0.01 mol/L or greater and 10 mol/L or less, a homogeneous liquid mixture including the raw material zeolite and the solution, in which the rubidium compound is dissolved in the solvent, can be obtained, and such a concentration is also advantageous in view of cost.

The amount of substance of the rubidium compound used in the liquid including the rubidium compound can be appropriately determined according to a ratio [(the valence × the amount of substance) of $Rb^+$ in the rubidium compound in the liquid including the rubidium compound/the quantity of acid sites of the raw material zeolite] of the product of the valence of the cation ($Rb^+$) in the rubidium compound and the amount of substance of the cation ($Rb^+$) in the rubidium compound relative to the quantity of acid sites of the raw material zeolite (i.e., the amount of substance of sites of the raw material zeolite capable of receiving cations ($Rb^+$)). In the case where two or more rubidium compounds are used, the valence of the cation ($Rb^+$) in the rubidium compound is a sum of valences of the cations ($Rb^+$) in the two or more rubidium compounds, and the amount of substance of the cation ($Rb^+$) in the rubidium compound is a sum of the amounts of substance of cations ($Rb^+$) in the two or more rubidium compounds.

The ratio [(the valence × the amount of substance) of Rb⁺ in the rubidium compound in the liquid including the rubidium compound/the quantity of acid sites of the raw material zeolite] is not particularly limited, and may be appropriately selected according to the intended purpose. The ratio [(the valence × the amount of substance) of Rb⁺ in the rubidium compound in the liquid including the rubidium compound/the quantity of acid sites of the raw material zeolite] is preferably 0.6 or greater and 50 or less, more preferably 0.6 or greater and 20 or less, and more preferably 1.0 or greater and 20 or less. When the ratio [(the valence × the amount of substance) of Rb⁺ in the rubidium compound in the liquid including the rubidium compound/the quantity of acid sites of the raw material zeolite] is 0.6 or greater, cations (Rb⁺) of the rubidium nitrate can be sufficiently imparted to the sites of the raw material zeolite capable of receiving cations. When the ratio [(the valence × the amount of substance) of Rb⁺ in the rubidium compound in the liquid including the rubidium compound/the quantity of acid sites of the raw material zeolite] is 50 or less, it is advantageous in view of cost.

The quantity of acid sites in the raw material zeolite can be determined, for example, by a method in which an amount of base adsorbed on the raw material zeolite is measured by temperature programmed deposition (TPD) using an appropriate base (e.g., ammonia).

A method of mixing the raw material zeolite and the liquid including the rubidium compound to obtain the liquid mixture is not particularly limited, and may be appropriately selected according to the intended purpose. Examples of the method include a method in which the raw material zeolite is immersed in a solution in which the rubidium compound is dissolved in a solvent.

In the mixing step, a range (range of yz/x (mmol/g)) obtained by dividing the numerical value, which is obtained by multiplying y and z, by x is not limited, where x (g) is a mass of at least one selected from the group consisting of the BEA zeolite and the MOR zeolite, y is a molar ratio [SiO₂/Al₂O₃] of SiO₂ to Al₂O₃ of at least one selected from the group consisting of the BEA zeolite including Rb⁺ and the MOR zeolite including Rb⁺, z (mmol) is the number of moles of the rubidium compound. The range may be appropriately selected according to the intended purpose. The mixing step preferably satisfies $1 \leq yz/x \leq 5{,}000$. In the case of the BEA zeolite, the mixing step more preferably satisfies $50 \leq yz/x \leq 5{,}000$, yet more preferably $200 \leq yz/x \leq 1{,}000$, yet more preferably $300 \leq yz/x \leq 1{,}000$, and particularly preferably $400 \leq yz/x \leq 800$. In the case of the MOR zeolite, the mixing step more preferably satisfies $1 \leq yz/x \leq 100$, and yet more preferably $8 \leq yz/x \leq 20$. Since the above ranges are satisfied, an effect of improving the useful component yield owing to Rb⁺ being included in at least one selected from the group consisting of the BEA zeolite and the MOR zeolite can be sufficiently exhibited, and it is advantageous in view of reduction in labor for preparing a zeolite and a raw material cost.

---Stirring step---

The stirring step is a step of stirring the liquid mixture including the raw material zeolite and the liquid including the rubidium compound to obtain the solution-treated zeolite.

A method of stirring the liquid mixture is not particularly limited, and any method known in the related art can be used.

The stirring time of the liquid mixture is not particularly limited, and may be appropriately determined according to the concentration of the rubidium compound in the liquid including the rubidium compound. The stirring time is preferably 0.5 hours to 48 hours, more preferably 1 hour to 24 hours, and yet more preferably 6 hours to 15 hours at room temperature in the air. When the stirring time is 0.5 hours or longer, cations included in the rubidium compound can be sufficiently imparted to the raw material zeolite. When the stirring time is 48 hours or shorter, contamination with substances included in the air, or uneven distribution of cations due to a change in the concentration of the liquid mixture can be prevented.

The solution-treated zeolite, which is obtained by performing the stirring step, is preferably filtered, for example, by a method of performing natural filtration, a method of performing suction filtration, or the like, and washed. Washing of the solution-treated zeolite can be performed, for example, by a method of passing a solvent, which is the same solvent as the solvent used in the solution in which the rubidium compound is dissolved in the solvent, through the solution-treated zeolite collected by the filtration.

---Evaporation drying step---

The evaporation drying step is a step of drying the zeolite by evaporation without filtration of the solution-treated zeolite. Since the evaporation drying step is included, cation exchange can progress further.

A method for the evaporation drying is not particularly limited, and may be appropriately selected according to a type of the solvent included in the liquid mixture. In the case where the solvent is water, examples of the method for the evaporation drying include a method in which the solution-treated zeolite is left to stand in the air within a room temperature environment for an appropriate time. In this case, the time for leaving the solution-treated zeolite to stand is not particularly limited as long as the solvent can be removed, but is preferably 2 days to 14 days, more preferably 3 days to 12 days, and yet more preferably 4 days to 6 days.

The evaporation drying step may be performed by a method in which the solution-treated zeolite is heated in the air for an appropriate time within an oven set at a temperature that is equal to or higher than room temperature and equal to or lower than a boiling point of the solvent, and is a temperature at which bumping of the solvent does not occur. According to this method, the time required to complete evaporation drying can be shortened. The time of heating in the oven can be appropriately adjusted according to the heating temperature. For example, in the case where the solvent is water, the heating may be performed at a temperature of approximately 80°C for 5 hours to 10 hours, or at a temperature of approximately 50°C for 40 hours to 60 hours.

In addition, the solution-treated zeolite recovered by evaporation drying is preferably washed with a same solvent as the solvent used for preparation of the liquid including the rubidium compound, followed by being subjected to a calcination step for removing an excess rubidium compound that is assumed to be deposited on the surface of the solution-treated zeolite.

---Calcination step---

The calcination step is a step of calcining the zeolite obtained after the mixing step, the stirring step, or the evaporation drying step.

In the case where an acidic zeolite is used as the raw material zeolite, as the calcination step is performed, protons at the acid sites of the raw material zeolite and nitric acid ions derived from the rubidium compound, which still remain even after the mixing step and the stirring step, are evaporated as water and nitrogen dioxide, thereby further facilitating incorporation of cations into the zeolite. In addition, the solvent remaining even after the filtration or the evaporation drying step can be almost completely removed.

The conditions and temperature of calcination of the solution-treated zeolite in the calcination step are not particularly limited, but are preferably 300°C to 600°C in the air, and more preferably 450°C to 550°C in the air.

The calcination time of the solution-treated zeolite in the calcination step is not particularly limited, but is preferably 1 hour to 20 hours, and more preferably 6 hours to 15 hours.

The pore size of the BEA zeolite including $Rb^+$ and the pore size of the MOR zeolite including $Rb^+$ can be controlled by varying the conditions of the mixing step and the stirring step. It is assumed that, as the BET specific area is smaller, a pore size of a resultant zeolite is smaller. Solid catalysts having different BET specific surface areas can be produced by changing at least one condition selected from the group consisting of: the ratio [(the valence $\times$ the amount of substance) of $Rb^+$ in the rubidium compound in the liquid including the rubidium compound/the quantity of acid sites of the raw material zeolite] of a product of the valence of the cation in the rubidium compound in the solution and the amount of substance of the cation in the rubidium compound to the amount of substance of sites of the raw material zeolite capable of receiving cations (the quantity of acid sites of the raw material zeolite); the concentration of the rubidium compound in a solution obtained by dissolving the rubidium compound in a solvent; and the stirring time of the liquid mixture.

<<Plastic>>

The plastic is not particularly limited, and may be appropriately selected according to the intended purpose. From the viewpoint of reduction in the environmental loads, the plastic preferably includes plastic waste, more preferably polyolefin, and yet more preferably at least one selected from the group consisting of polyethylene (PE), polypropylene (PP), and polystyrene (PS), which are commonly used in drink and food containers, wrapping materials, molded articles, films, and the like.

In addition to the above-described plastic materials, the plastic may further include other plastic materials, such as polyethylene terephthalate (PET), polybutylene terephthalate (PBT), polyethylene naphthalate (PEN), polycarbonate (PC), polyvinyl chloride, polyvinylidene chloride, chlorinated polyethylene, polyamide, polyurethane, an acrylonitrile-butadiene-styrene copolymer (ABS), polymethyl methacrylate, and the like.

In the case where the plastic is plastic waste, a composition ratio of the plastic waste is preferably 20 percent by mass to 40 percent by mass of PE : 20 percent by mass to 40 percent by mass of PP : 10 percent by mass to 30 percent by mass of PS.

An amount of the other plastic materials in the plastic is not particularly limited, and may be appropriately selected according to a type of plastic waste. In view of a yield of olefins with 2 to 5 carbon atoms, the amount of the other plastic materials is preferably 50 percent by mass or less, more preferably 45 percent by mass or less, and yet more preferably 40 percent by mass or less, relative to a total mass of the plastic.

The plastic may be a plastic composition that further includes a material or additive other than the plastic. In the case where the plastic composition is a plastic waste composition, examples of the material other than the plastic include paper and metals.

An amount of the material or additive other than plastic in the plastic composition is not particularly limited.

The decomposition step is preferably performed by heating in view of favorable decomposition efficiency.

A reaction vessel in which the decomposition step is performed is not particularly limited, and may be appropriately selected according to the intended purpose. Examples of the reaction vessel include a batch reactor, a fixed bed reactor, and a fluidized bed reactor.

The temperature at which the decomposition step is performed (may be referred to as a "decomposition temperature" hereinafter) is not particularly limited, and may be appropriately selected according to the intended purpose. The decomposition temperature is preferably 300°C or higher and 1,100°C or lower, and more preferably 300°C or higher and 600°C or lower. When the decomposition temperature is 300°C or higher and 1,100°C or lower, the catalyst is easily activated, and a favorable yield of olefins with 2 to 5 carbon atoms is achieved.

The time for performing decomposition at the decomposition temperature (may be referred to as a "decomposition time" hereinafter) is not particularly limited, and may be appropriately selected according to a reaction system or the like. For example, in the case where the decomposition is performed using a sealed batch reactor, the decomposition time is preferably 1 hour or longer and 20 hours or shorter, and more preferably 5 hours or longer and 10 hours or shorter. When the decomposition time is 1 hour or longer, the plastic can be suitably decomposed, and a favorable yield of olefins with 2 to 5 carbon atoms is achieved. When the decomposition time is 20 hours or shorter, the decomposition is performed efficiently.

The atmosphere in which the decomposition is performed is not particularly limited, and may be appropriately selected according to the intended purpose. The decomposition is preferably performed in the presence of an inert gas.

The inert gas is not particularly limited, and may be appropriately selected according to the intended purpose. Examples of the inert gas include a nitrogen gas and an argon gas. The above gases may be used alone or in combination.

In order to completely fluidize the plastic, part of the plastic may be vaporized in the decomposition step. In case of industrial waste, a relatively uniform plastic waste material that is formed of specific plastic components and has a molecular weight distribution within a certain range may be obtained. In such a case, processing conditions of the decomposition step are preferably adjusted according to types of the plastic components constituting the plastic waste material and the molecular weight of the plastic waste material. When inorganic materials are separated from a plastic waste material that is general waste, a separation device of a laboratory scale may be used, but the separation can also be performed at an industrial level in various plants, such as an oil refinery and the like.

<Other steps>

The other steps in the method for producing the olefin-containing composition are not particularly limited, and may be appropriately selected according to the intended purpose. Examples of the other steps include a pretreatment step of the plastic and a recovery step of a volatile component obtained in the decomposition step. In addition, the above-described mixing step, stirring step, and calcination step for producing at least one selected from the group consisting of the BEA zeolite including $Rb^+$ and the MOR zeolite including $Rb^+$ may be included.

<<Pretreatment step>>

The pretreatment step is a step of pretreating the plastic before subjecting the plastic to the decomposition step. Since the plastic is transformed into a form or state that is easily decomposed, the plastic can be decomposed more efficiently.

Examples of the pretreatment include pulverization of the plastic, palletization (chipping) of the pulverized product of the plastic, and melting of the plastic.

The melting of the plastic is preferably performed at lower than 300°C in the absence of the zeolite.

The pulverized product of the plastic is not particularly limited, and may be appropriately selected according to the intended purpose. Examples of the pulverized product include powders and flakes.

The method of obtaining the pulverized product of the plastic is not particularly limited, and may be appropriately selected from methods known in the related art. Examples of the method include a method in which the plastic is pulverized by a pulverizer to obtain a powder or flakes.

The method of pelletizing (chipping) the pulverized product is also not limited, and may be appropriately selected from methods known in the related art. Examples of the method include a method in which, after melting and extruding the pulverized product, the strand-shaped melt-extrusion product is cut to obtain the raw material in the form of chips.

The plastic can be supplied to the decomposition step in the melted state. The method of melting the plastic is not particularly limited, and may be appropriately selected from methods known in the art. Examples of the method include a method in which the plastic is continuously supplied to the decomposition step using a melt-extruder.

<<Volatile component recovery step>>

The volatile component recovery step is a step of recovering the volatile component obtained in the decomposition step.

Inclusion of the volatile component recovery step is advantageous because, when a volatile component is generated in the decomposition step, the volatile component can be collected or removed according to usefulness of the volatile component. For example, in the case where the plastic is polystyrene, styrene monomers generated in the decomposition step can be vaporized and recovered. The recovered styrene monomers are advantageous because the recovered styrene monomers can be reused in production of polystyrene or the like. In the case where the plastic material includes chlorine-containing plastic, such as polyvinyl chloride and polyvinylidene chloride, hydrogen chloride generated when the chlorine-containing plastic is heated in the decomposition step is preferably vaporized and removed. Separately from the decomposition step, the plastic material may be heated at a low temperature in advance to vaporize hydrogen chloride, followed by subjecting the resultant plastic material to the decomposition step. In this case, the temperature for the heating at the low temperature is preferably 100°C or higher and lower than 300°C. Thus, after the plastic is fluidized, the chlorine content of the fluidized olefin-containing composition can be reduced.

The method of recovering the volatile component is not particularly limited, and may be appropriately selected from methods known in the related art.

According to the above method for producing the olefin-containing composition, a raw material, which achieves an excellent useful component yield, and is suitably used for chemical recycling, can be obtained. In the case where the BEA zeolite including $Rb^+$ is used as the zeolite, it is advantageous especially in view of an excellent useful aromatic yield. It has been known that, as an amount of an aromatic component is increased, a residue is generally likely to be generated (see, for example, the paragraph [0003] of Japanese translation of International Application Publication No. 2023-504126). However, use of the BEA zeolite including $Rb^+$ is advantageous in view of a high yield of useful aromatics and unlikeliness to generate a residue.

(Catalyst for producing olefin-containing composition)

A catalyst for producing an olefin-containing composition according to one embodiment of the present disclosure is a catalyst used in production of an olefin-containing composition including an olefin with 2 to 5 carbon atoms. The catalyst for producing the olefin-containing composition includes at least one selected from the group consisting of a BEA zeolite including $Rb^+$ and a MOR zeolite including $Rb^+$, and may further include other components, as necessary.

Examples of a raw material of the olefin-containing composition include plastic.

<BEA zeolite including $Rb^+$ and MOR zeolite including $Rb^+$>

The BEA zeolite including $Rb^+$ and the MOR zeolite including $Rb^+$ are described in the above section of (Method for producing olefin-containing composition).

The amount of at least one selected from the group consisting of the BEA zeolite including $Rb^+$ and the MOR zeolite including $Rb^+$ in the catalyst for producing the olefin-containing composition is not particularly limited as long as the effects of the present embodiment are not adversely affected, and may be appropriately selected according to the intended purpose. The catalyst for producing the olefin-containing composition may be at least one selected from the group consisting of the BEA zeolite including $Rb^+$ and the MOR zeolite including $Rb^+$ itself (i.e., the amount of at least one selected from the group consisting of the BEA zeolite including $Rb^+$ and the MOR zeolite including $Rb^+$ being 100 percent by mass).

<Other components>

The other components in the catalyst for producing the olefin-containing composition are not particularly limited. Examples of the other components include a pH adjuster, a preservative or fungicide, a caking additive (binder), and a granulation aid.

The amount of the other components in the catalyst for producing the olefin-containing composition is not particularly limited as long as the effects of the present embodiment are not adversely affected, and may be appropriately selected according to the intended purpose.

The catalyst for producing the olefin-containing composition is a catalyst, specifically, a catalyst including at least one selected from the group consisting of the BEA zeolite including $Rb^+$ and the MOR zeolite including $Rb^+$, which is used in production of an olefin-containing composition including an olefin with 2 to 5 carbon atoms. Accordingly, the method of using the catalyst including at least one selected from the group consisting of a beta zeolite including $Rb^+$ and a mordenite zeolite including $Rb^+$ in production of an olefin-containing composition including an olefin with 2 to 5 carbon atoms is also included in the scope of the present disclosure.

Examples

The embodiments of the present disclosure will be concretely described hereinafter through Production Examples, Comparative Production Examples, Examples, and Comparative Examples. However, the embodiments of the present disclosure are not limited by Test Examples, Production Examples, Comparative Production Examples, Examples, and Comparative Examples in any way. In the following description, "%" denotes "percent by mass" unless otherwise specified.

(Production Example 1)

<Processing step>

Pure water was added to 0.39 g (mass) (2.6 mmol) of rubidium nitrate (RbNO$_3$, purity: 95.0% or greater, manufactured by KANTO CHEMICAL CO., INC.), and the rubidium nitrate was completely dissolved in the pure water, thereby preparing a 0.26 mol/L rubidium nitrate aqueous solution. In a glass container, 3.0 g of a beta (BEA) zeolite (product name: HSZ-980HOA, manufactured by Tosoh Corporation, molar ratio [SiO$_2$/Al$_2$O$_3$]: 40, BET specific surface area: 500 m$^2$/g) and the entire amount of the rubidium nitrate aqueous solution were mixed, and the mixture was stirred at room temperature (20°C ± 5°C) for 12 hours in the air, thereby obtaining a reaction product.

<Calcination step>

After the processing step, the reaction product was left to stand at room temperature for 5 days in the air to dry the reaction product by evaporation, thereby obtaining a solid product. After washing the obtained solid product with water, the washed solid product was transferred into a porcelain crucible, and was calcined in an electric furnace set at 500°C for 8 hours in the air, thereby obtaining a solid catalyst of Production Example 1.

(Production Example 2)

A solid catalyst of Production Example 2 was obtained in the same manner as in Production Example 1, except that the preparation of the 0.26 mol/L rubidium nitrate aqueous solution performed by adding pure water to and completely dissolving 0.39 g (mass) (2.6 mmol) of the rubidium nitrate was changed to preparation of a 0.23 mol/L rubidium nitrate aqueous solution performed by adding pure water to and completely dissolving 0.34 g (mass) (2.3 mmol) of rubidium nitrate, and 3.0 g of the beta zeolite was changed to 3.0 g of a mordenite (MOR) zeolite (product name: HSZ-640HOA, manufactured by Tosoh Corporation, molar ratio [SiO$_2$/Al$_2$O$_3$]: 18, BET specific surface area: 380 m$^2$/g).

(Production Example 3)

A solid catalyst of Production Example 3 was obtained in the same manner as in Production Example 1, except that the preparation of the 0.26 mol/L rubidium nitrate aqueous solution performed by adding pure water to and completely dissolving 0.39 g (mass) (2.6 mmol) of the rubidium nitrate was changed to preparation of a 0.12 mol/L rubidium nitrate aqueous solution performed by adding pure water to and completely dissolving 0.18 g (mass) (1.2 mmol) of rubidium nitrate.

(Production Example 4)

A solid catalyst of Production Example 4 was obtained in the same manner as in Production Example 1, except that the preparation of the 0.26 mol/L rubidium nitrate aqueous solution performed by adding pure water to and completely dissolving 0.39 g (mass) (2.6 mmol) of the rubidium nitrate was changed to preparation of a 0.35 mol/L rubidium nitrate aqueous solution performed by adding pure water to and completely dissolving 0.52 g (mass) (3.5 mmol) of rubidium nitrate.

(Production Example 5)

A solid catalyst of Production Example 5 was obtained in the same manner as in Production Example 1, except that the preparation of the 0.26 mol/L rubidium nitrate aqueous solution performed by adding pure water to and completely dissolving 0.39 g (mass) (2.6 mmol) of the rubidium nitrate was changed to preparation of a 0.47 mol/L rubidium aqueous solution performed by adding pure water to and completely dissolving 0.69 g (mass) (4.7 mmol) of rubidium nitrate.

(Comparative Production Example 1)

A solid catalyst of Comparative Production Example 1 was obtained in the same manner as in Production Example 1, except that the preparation of the 0.26 mol/L rubidium nitrate aqueous solution performed by adding pure water to and completely dissolving 0.39 g (mass) (2.6 mmol) of the rubidium nitrate was changed to preparation of a 0.26 mol/L sodium nitrate aqueous solution performed by adding pure water to and completely dissolving 0.22 g (mass) (2.6 mmol) of sodium nitrate (NaNO$_3$, purity: 95.0% or higher, manufactured by KANTO CHEMICAL CO., INC.).

(Comparative Production Example 2)

A solid catalyst of Comparative Production Example 2 was obtained in the same manner as in Production Example 2, except that the preparation of the 0.23 mol/L rubidium nitrate aqueous solution performed by adding pure water to and completely dissolving 0.34 g (mass) (2.3 mmol) of the rubidium nitrate was changed to preparation of a 0.23 mol/L cesium nitrate aqueous solution performed by adding pure water to and completely dissolving 0.45 g (mass) (2.3 mmol) of cesium nitrate (CsNO$_3$, purity: 95.0% or higher, manufactured by KANTO CHEMICAL CO., INC.).

(Comparative Production Example 3)

A solid catalyst of Production Example 2 was obtained in the same manner as in Production Example 1, except that the preparation of the 0.26 mol/L rubidium nitrate aqueous solution performed by adding pure water to and completely dissolving 0.39 g (mass) (2.6 mmol) of the rubidium nitrate was changed to preparation of a 0.37 mol/L rubidium nitrate aqueous solution performed by adding pure water to and completely dissolving 0.54 g (mass) (3.7 mmol) of rubidium nitrate, and 3.0 g of the beta zeolite was changed to 3.0 g of a faujasite (FAU) zeolite (product name: HSZ-350HUA, manufactured by Tosoh Corporation, molar ratio [SiO$_2$/Al$_2$O$_3$]: 10, BET specific surface area: 650 m$^2$/g).

<Measurement of quantity of acid sites of solid catalyst (zeolite)>

A quantity of acid sites of each of the BEA zeolite (product name: HSZ-980HOA), the MOR zeolite (product name: HSZ-640HOA), and the FAU zeolite (product name: HSZ-350HUA) (these may be collectively referred to as "raw material zeolites" hereinafter) that were used as the raw materials of the solid catalysts of Production Examples 1 to 5 and Comparative Production Examples 1 to 3, was analyzed by ammonia temperature programmed deposition (TPD) under the following measuring conditions.

[Measuring conditions of quantity of acid sites]

Device: BEL-CAT-BASIC (manufactured by MicrotracBEL Corp.)
Detector: quadrupole mass analyzer BEL-Mass (manufactured by MicrotracBEL Corp.)
Measurement target species: NH$_3$ (m/z = 16)
Pretreatment conditions: The pretreatment was performed in the order of 1 to 9 in Table 1 below under the conditions presented in Table 1 below.
Heating conditions: The temperature was elevated from 100°C to 850°C at 10°C/min, and kept at 850°C for 30 minutes. The flow rate of the He gas during heating was 30 mL/min.

[Table 1]

| Pretreatment order | Set temperature [°C] | Set time [min] | Flowing gas | Gas flow rate [mL/min] |
|---|---|---|---|---|
| 1 | 25 | 30 | He | 50 |
| 2 | 25 to 600 | 60 | He | 50 |
| 3 | 600 | 60 | He | 50 |
| 4 | 600 to 100 | 10 | He | 50 |
| 5 | 100 | 10 | He | 50 |
| 6 | 100 | 30 | 5%NH$_3$/95%He | 50 |

(continued)

| Pretreatment order | Set temperature [°C] | Set time [min] | Flowing gas | Gas flow rate [mL/min] |
|---|---|---|---|---|
| 7 | 100 | 30 | He | 50 |
| 8 | 100 | 30 | He (water vapor treatment) | 50 |
| 9 | 100 | 300 | He | 50 |

The quantity of acid sites of the BEA zeolite (product name: HSZ-980HOA) was 0.079 mmol/g, the quantity of acid sites of the MOR zeolite (product name: HSZ-640HOA) was 1.0 mmol/g, and the quantity of acid sites of the FAU zeolite (product name: HSZ-350HUA) was 1.7 mmol/g.

<Calculation of ratio [(valence $\times$ amount of substance) of cation in nitrate included in nitrate aqueous solution/ quantity of acid sites in raw material zeolite]>

The quantity of acid sites in the raw material zeolite used in the production of each of the solid catalysts of Production Examples 1 to 5 and Comparative Production Examples 1 to 3 corresponds to an amount of substance of the sites of the raw material zeolite capable of receiving cations. In addition, a product of a valence and an amount of substance of the cation ($Rb^+$, $Na^+$, or $Cs^+$) of the corresponding nitrate (rubidium nitrate, sodium nitrate, or cesium nitrate) included in each of the rubidium nitrate aqueous solutions prepared in Production Examples 1 to 5 and Comparative Production Example 3, the sodium nitrate aqueous solution prepared in Comparative Production Example 1, and the cesium nitrate aqueous solution prepared in Comparative Production Example 2 (these may be collectively referred to as "nitrate aqueous solutions" hereinafter) was determined. Subsequently, the ratio [(the valence $\times$ the amount of substance) of the cation in the nitrate included in the nitrate aqueous solution/the quantity of acid sites in the raw material zeolite] of (the valence $\times$ the amount of substance) of the cation in the nitrate included in the nitrate aqueous solution to the quantity of acid sites of the raw material zeolite was calculated. The results are presented in Tables 2-1 and 2-2 below.

[Evaluation of physical properties of solid catalyst]

For the solid catalysts (zeolites) obtained in Production Examples 1 to 5 and Comparative Production Examples 1 to 3, and the BEA zeolite (product name: HSZ-980HOA), the MOR zeolite (product name: HSZ-640HOA), and the FAU zeolite (product name: HSZ-350HUA) used as raw materials in production of the solid catalysts of Production Examples 1 to 5 and Comparative Production Examples 1 to 3, a crystal system, BET specific surface area, molar ratio [$SiO_2/Al_2O_3$], and nitrate concentration in the aqueous solution were analyzed by the following methods. The results are presented in Tables 2-1 and 2-2 below.

In addition, in Tables 2-1 and 2-2 below, a cationic crystal radius for ions included in each of the solid catalysts (zeolites) obtained in Production Examples 1 to 5 and Comparative Production Examples 1 to 3, and the BEA zeolite (product name: HSZ-980HOA), the MOR zeolite (product name: HSZ-640HOA), and the FAU zeolite (product name: HSZ-350HUA) used as raw materials in the production of the solid catalysts of Production Examples 1 to 5 and Comparative Production Examples 1 to 3, indicates a value of an ionic radius determined by R. D. Shannon and C. T. Prewitt, and the cationic element electronegativity indicates a value of Pauling electronegativity.

<Analysis of crystal system of solid catalyst (zeolite)>

Crystal systems of the solid catalysts (zeolites) obtained in Production Examples 1 to 5 and Comparative Production Examples 1 to 3, and the BEA zeolite (product name: HSZ-980HOA), the MOR zeolite (product name: HSZ-640HOA), and the FAU zeolite (product name: HSZ-350HUA) used as the raw materials in the production of the solid catalysts of Production Examples 1 to 5 and Comparative Production Examples 1 to 3, were analyzed by X-ray diffraction (XRD) under the following measuring conditions.

[X-ray diffraction measuring conditions]

Device: X'Pert Pro MPD (manufactured by Pnalytical) X-ray source: CuKα rays
Incident X-ray side filter: 10 mm brass mask
Detector-side filter: Ni filter

Incident-side slit: Sollerslit 0.04 rad ASS1/8°
Detector-side slit: ASS 5.0 mm Sollerslit 0.04 rad
Detector: PIXel1D
Measurement method: reflection spectroscopy
Operation range (measuring range): 2θ = 5° to 120°
Step width: 0.006565°
Counting time: 78.795 sec/step

<Measurement of BET specific surface area of solid catalyst (zeolite)>

BET specific surface areas of the solid catalysts (zeolites) obtained in Production Examples 1 to 5 and Comparative Production Examples 1 to 3, and the BEA zeolite (product name: HSZ-980HOA), the MOR zeolite (product name: HSZ-640HOA), and the FAU zeolite (product name: HSZ-350HUA) used as the raw materials in the production of the solid catalysts of Production Examples 1 to 5 and Comparative Production Examples 1 to 3, were measured according to ISO 9277:2010 "Determination of the specific surface area of powder (solids) by gas adsorption".

Specifically, the specific surface area was determined by measuring the zeolite by a specific surface area analyzer (BELSORP (registered trademark) MAX II, manufactured by MicrotracBEL Corp.) at a liquid nitrogen temperature using a nitrogen molecule as a probe according to ISO 9277:2010, and analyzing the result according to the BET method.

<Measurement of molar ratio [$SiO_2/Al_2O_3$] of solid catalyst (zeolite)>

Each of the solid catalysts (zeolites) obtained in Production Examples 1 to 5 and Comparative Production Examples 1 to 3, and the BEA zeolite (product name: HSZ-980HOA), the MOR zeolite (product name: HSZ-640HOA), and the FAU zeolite (product name: HSZ-350HUA) used as the raw materials in the production of the solid catalysts of Production Examples 1 to 5 and Comparative Production Examples 1 to 3, was weighed, the weighed zeolite was completely dissolved in an aqueous solution including nitric acid (industrial electronic EL nitric acid 1.38, manufactured by KANTO CHEMICAL CO., INC., purity: 60.0% to 61.0%) and hydrofluoric acid (ultrapure reagent Ultrapur™-100, manufactured by KANTO CHEMICAL CO., INC., purity: 46.0% to 51.0%), and the volume of the resultant solution was fixed, thereby preparing a measurement sample. Amounts of Si and Al in the measurement sample were measured by ICP optical emission spectrometer (PlasmaQuant PQ 9000, manufactured by Analytik Jena AG), and a molar ratio [$SiO_2/Al_2O_3$] was calculated according to the following equation based on the number of moles of Si and Al calculated from the amounts of Si and Al.

Molar ratio [$SiO_2/Al_2O_3$] = 2 × (number of moles of Si)/(number of moles of Al)

<Measurement of nitrate concentration in solution>

The nitrate concentration in each of the nitrate aqueous solutions used in the processing step of Production Examples 1 to 5 and Comparative Production Examples 1 to 3 was determined by molar calculation, assuming that there was no change in the liquid amount due to mixing.

[Table 2-1]

| | Production Ex. 1 | Production Ex. 2 | Production Ex. 3 | Production Ex. 4 | Production Ex. 5 |
|---|---|---|---|---|---|
| | | | | | |
| Type of solid catalyst | HSZ-980H-OA | HSZ-640H-OA | HSZ-980H-OA | HSZ-980H-OA | HSZ-980H-OA |
| Type of nitrate | $RbNO_3$ | $RbNO_3$ | $RbNO_3$ | $RbNO_3$ | $RbNO_3$ |
| Included ion | $Rb^+$ | $Rb^+$ | $Rb^+$ | $Rb^+$ | $Rb^+$ |
| | | | | | |
| Crystal system | BEA | MOR | BEA | BEA | BEA |
| Cationic crystal radius [pm] | 166 | 166 | 166 | 166 | 166 |
| Cationic element electronegativity | 0.82 | 0.82 | 0.82 | 0.82 | 0.82 |

(continued)

|  | Production Ex. 1 | Production Ex. 2 | Production Ex. 3 | Production Ex. 4 | Production Ex. 5 |
|---|---|---|---|---|---|
| Ion-exchange anion | $NO_3^-$ | $NO_3^-$ | $NO_3^-$ | $NO_3^-$ | $NO_3^-$ |
| BET specific surface area [m$^2$/g] | 438 | 372 | 446 | 362 | 322 |
| Mass of BEA raw material zeolite (x) [g] | 3 | - | 3 | 3 | 3 |
| Mass of MOR raw material zeolite (x) [g] | - | 3 | - | - | - |
| Mass of FAU raw material zeolite (x) [g] | - | - | - | - | - |
| Molar ratio [$SiO_2/Al_2O_3$] of raw material zeolite (y) | 500 | 18 | 500 | 500 | 500 |
| Number of moles of nitrate (z) [mmol] | 2.6 | 2.3 | 1.2 | 3.5 | 4.7 |
| Nitrate concentration in nitrate aqueous solution [mol/L] | 0.26 | 0.23 | 0.12 | 0.35 | 0.47 |
| yz/x | 433 | 14 | 200 | 583 | 783 |
| Ratio [ (valence × amount of substance) of cation in nitrate included in nitrate aqueous solution/quantity of acid sites of raw material zeolite] | 11.00 | 0.77 | 5.00 | 15.00 | 20.00 |

[Table 2-2]

|  | Comp. Pro. Ex. 1 | Comp. Pro. Ex. 2 | Comp. Pro. Ex. 3 | 980HOA | 640HOA | 350HUA |
|---|---|---|---|---|---|---|
|  |  |  |  |  |  |  |
| Type of solid catalyst | HS-Z-980H-OA | HS-Z-640H-OA | HS-Z-350H-UA | HS-Z-980H-OA | HS-Z-640H-OA | HS-Z-350H-UA |
| Type of nitrate | $NaNO_3$ | $CsNO_3$ | $RbNO_3$ | - | - | - |
| Included ion | $Na^+$ | $Cs^+$ | $Rb^+$ | H' | $H^+$ | $H^+$ |
|  |  |  |  |  |  |  |
| Crystal system | BEA | MOR | FAU | BEA | MOR | FAU |
| Cationic crystal radius [pm] | 116 | 181 | 166 | -4 | -4 | -4 |
| Cationic element electronegativity | 0.93 | 0.79 | 0.82 | 2.20 | 2.20 | 2.20 |
| Ion-exchange anion | $NO_3–$ | $NO_3–$ | $NO_3–$ | NA | NA | NA |
| BET specific surface area [m$^2$/g] | 328 | 200 | 553 | 500 | 380 | 650 |
| Mass of BEA raw material zeolite (x) [g] | 3 | - | - | - | - | - |
| Mass of MOR raw material zeolite (x) [g] | - | 3 | - | - | - | - |
| Mass of FAU raw material zeolite (x) [g] | - | - | 3 | - | - | 3 |
| Molar ratio [$SiO_2/Al_2O_3$] of raw material zeolite (y) | 500 | 18 | 10 | 500 | 18 | 10 |
| Number of moles of nitrate (z) [mmol] | 2.6 | 2.3 | 3.7 | - | - | - |
| Nitrate concentration of nitrate aqueous solution [mol/L] | 0.26 | 0.23 | 0.37 | - | - | - |
| yz/x | 433 | 14 | 12 | - | - | - |

(continued)

|  | Comp. Pro. Ex. 1 | Comp. Pro. Ex. 2 | Comp. Pro. Ex. 3 | 980HOA | 640HOA | 350HUA |
|---|---|---|---|---|---|---|
| Ratio [ (valence × amount of substance) of cation in nitrate included in nitrate aqueous solution/quantity o acid sites of raw material zeolite] | 11.00 | 0.77 | 11.00 | - | - | - |

(Example 1)

Decomposition of a plastic mixture was performed in the presence of the solid catalyst (may be referred to as the "Rb$^+$-substituted BEA zeolite" hereinafter) obtained in Production Example 1 according to the following method.

The mixture including 0.3 g of polyethylene (manufactured by Sigma-Aldrich, the number average molecular weight: 1,700, the weight average molecular weight: 4,000), 0.3 g of polypropylene (manufactured by Sigma-Aldrich, the number average molecular weight: 5,000, the weight average molecular weight: 12,000), 0.3 g of polystyrene (manufactured by Sigma-Aldrich, the weight average molecular weight: 35,000), and 0.2 g of the solid catalyst of Production Example 1 was placed in an inner cylinder formed of quartz. The inner cylinder was sealed in a high-temperature and high-pressure reaction vessel (Model 4790, formed of SUS-316, 100 mL in volume, manufactured by Parr Instrument Company) together with a nitrogen gas at atmospheric pressure, and was heated to an internal temperature of 450°C. The internal temperature was kept for 8 hours. After leaving the resultant product to cool, the gaseous product was recovered, and 10 mL of tetrahydrofuran (THF) (manufactured by KANTO CHEMICAL CO., INC., high-performance liquid chromatography reagent, no stabilizer added) was added to the residue, followed by sufficiently mixing the resultant mixture at room temperature. The solid product was separated by filtration, and the solid residue was washed with a small amount of THF twice. In this manner, a product soluble in THF (THF-soluble product) was extracted in the THF solution obtained by combining the filtrate and the washing liquid.

(Example 2)

A gaseous product and a THF-soluble product were extracted in the same manner as in Example 1, except that the solid catalyst of Production Example 1 was changed to the solid catalyst of Production Example 2 (may be referred to as an "Rb$^+$-substituted MOR zeolite" hereinafter).

(Example 3)

A gaseous product and a THF-soluble product were extracted in the same manner as in Example 1, except that the solid catalyst of Production Example 1 was changed to the solid catalyst of Production Example 3 (may be referred to as an "Rb$^+$-substituted BEA zeolite" hereinafter).

(Example 4)

A gaseous product and a THF-soluble product were extracted in the same manner as in Example 1, except that the solid catalyst of Production Example 1 was changed to the solid catalyst of Production Example 4 (may be referred to as an "Rb$^+$-substituted BEA zeolite" hereinafter).

(Example 5)

A gaseous product and a THF-soluble product were extracted in the same manner as in Example 1, except that the solid catalyst of Production Example 1 was changed to the solid catalyst of Production Example 5 (may be referred to as an "Rb$^+$-substituted BEA zeolite" hereinafter).

(Comparative Example 1)

A gaseous product and a THF-soluble product were extracted in the same manner as in Example 1, except that the solid catalyst of Production Example 1 was changed to the solid catalyst of Comparative Production Example 1 (may be referred to as an "Na$^+$-substituted BEA zeolite" hereinafter).

(Comparative Example 2)

A gaseous product and a THF-soluble product were extracted in the same manner as in Example 1, except that the solid catalyst of Production Example 1 was changed to a BEA zeolite (HSZ-980HOA).

(Comparative Example 3)

A gaseous product and a THF-soluble product were extracted in the same manner as in Example 1, except that the solid catalyst of Production Example 1 was changed to the solid catalyst of Comparative Production Example 2 (may be referred to as a "Cs$^+$-substituted zeolite" hereinafter).

(Comparative Example 4)

A gaseous product and a THF-soluble product were extracted in the same manner as in Example 1, except that the solid catalyst of Production Example 1 was changed to an MOR zeolite (product name: HSZ-640HOA).

(Comparative Example 5)

A gaseous product and a THF-soluble product were extracted in the same manner as in Example 1, except that the solid catalyst of Production Example 1 was changed to the solid catalyst of Comparative Production Example 3 (may be referred to as an "Ra$^+$-substituted FAU zeolite" hereinafter).

(Comparative Example 6)

A gaseous product and a THF-soluble product were extracted in the same manner as in Example 1, except that the solid catalyst of Production Example 1 was changed to an FAU zeolite (product name: HSZ-350HUA).

[Evaluation of plastic degradability]

To each of the gaseous products recovered in Examples 1 to 5 and Comparative Examples 1 to 6, 38 mg of cyclopentane (reagent, manufactured by Tokyo Chemical industry Co., Ltd., purity: 98.0% or higher) was added as an internal standard substance, thereby preparing an analysis sample. In addition, to each of the THF-soluble products recovered in Examples 1 to 5 and Comparative Examples 1 to 6, 0.12 g of t-butylbenzene (reagent, manufactured by Tokyo Chemical industry Co., Ltd., purity: 98.0% or higher) was added as an internal standard substance, thereby preparing an analysis sample.
Each of these analysis samples was analyzed by a gas chromatography (GC) device equipped with a flame ionization detector under the following analysis conditions, to quantify each component based on a ratio between the peak area of each component and the peak area of the internal standard substance. The results are presented in Tables 3-1 and 3-2 below.

<<GC analysis conditions of gaseous product>> Device: Nexis GC-2030 (manufactured by Shimadzu Corporation)

Column: Rt-Alumina BOND (diameter: 0.32 mm, length: 30 m, manufactured by Restek)
Carrier gas type: Ar
Flow rate of carrier gas: 360 mL/min
Injection temperature: 200°C
Sample injection amount: 1 mL
Split ratio: 1/200
Column temperature: Setting a heating program to heat at 120°C (9 minutes), elevate the temperature (10 °C/min), and heat at 200°C (30 minutes)
Detector: flame ionization detector (FID)
Detector temperature: 200°C

<<GC analysis conditions of THF-soluble product>>

Device: Nexis GC-2030 (manufactured by Shimadzu Corporation)
Column: DB-1 (diameter: 0.25 mm, length: 30 m, manufactured by Agilent Technology)
Carrier gas type: He
Flow rate of carrier gas: 97 mL/min
Injection temperature: 350°C
Sample injection amount: 1 μL
Split ratio: 1/50
Column temperature: Setting a heating program to heat at 35°C (10 minutes), elevate the temperature (5 °C/min), and heat at 350°C (10 minutes)
Detector: flame ionization detector (FID)
Detector temperature: 350°C

In Tables 3-1 and 3-2 below, the "yield of useful components" encompasses a ratio of the total number of moles of carbon atoms included in olefins with 2 to 5 carbon atoms and useful aromatics in the product (useful components) to the number of moles of the carbon atoms included in the raw materials. The "useful components" encompass ethylene, propylene, olefins with 4 carbon atoms (1-butene, cis-2-butene, trans-2-butene, and 2-methylpropane), olefins with 5 carbon atoms (1-pentene, cis-2-pentene, trans-2-pentene, 2-methyl-1-butene, and 2-methyl-2-butene), and useful aromatics (benzene, toluene, ethyl benzene, three positional isomers of xylene (p-xylene, m-xylene, and o-xylene), styrene, and cumene). Moreover, the "yield of useful aromatics" encompasses a ratio of a total number of moles of carbon atoms included in the useful aromatics in the product (useful components) relative to the number of moles of the carbon atoms included in the raw materials. The "useful aromatics" encompass benzene, toluene, ethyl benzene, three positional isomers of xylene (p-xylene, m-xylene, and o-xylene), styrene, and cumene.

[Quantification of reaction residue]

The solid product remaining after extracting the THF-soluble product from the residue in the reaction vessel in each of Examples 1 to 5 and Comparative Examples 1 to 6 was dried, and the mass A of the dried solid product was measured. Separately, the mass B of the reaction vessel before the reaction, and the mass C of the reaction vessel that was emptied and dried after the reaction were measured, and the difference (mass C - mass B) between the mass B and the mass C was determined. A sum (mass A + (mass C - mass B)) of the mass A of the dried solid product and the difference (mass C - mass B) in the mass of the reaction vessel before and after the reaction was determined as a mass of the reaction residue. The number of moles was determined, assuming that the entire reaction residue was composed of carbons, and a ratio [the number of moles of carbon atoms in the reaction residue/the number of carbon atoms in the raw materials] of the number of moles of the carbon atoms in the reaction residue to the number of moles of carbon atoms in the raw materials was determined as a yield of the reaction residue. The results are presented in Tables 3-1 and 3-2 below.
[Table 3-1]

|  | | Ex. 1 | Ex. 2 | Ex. 3 | Ex. 4 | Ex. 5 |
|---|---|---|---|---|---|---|
| Solid catalyst | | $Rb^+$-substituted BEA zeolite (Pro. Ex. 1) | $Rb^+$-substituted MOR zeolite (Pro. Ex. 2) | $Rb^+$-substituted BEA zeolite (Pro. Ex. 3) | $Rb^+$-substituted BEA zeolite (Pro. Ex. 4) | $Rb^+$-substituted BEA zeolite (Pro. Ex. 5) |
| Yield [%] (based on carbon atoms) | Total of useful components | 44.39 | 41.61 | 35.21 | 48.76 | 41.87 |
| | Useful aromatics | 16.08 | 7.96 | 11.96 | 19.03 | 15.78 |
| Yield of reaction residue (ratio [number of moles of carbon atoms in reaction residue/number of moles of carbon atoms in raw materials]) | | 0.00 | 0.00 | 0.00 | 0.00 | 0.00 |

[Table 3-2]

| | | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Solid catalyst | | $Na^+$-substituted BEA zeolite (Comp. Pro. Ex. 1) | HS-Z-980H-OA | $Cs^+$-substituted MOR zeolite (Comp. Pro. Ex. 2) | HS-Z-640H-OA | $Rb^+$-substituted FAU zeolite (Comp. Pro. Ex. 3) | HS-Z-350H-UA |
| Yield [%] (based on carbon atoms) | Total of useful components | 25.36 | 19.10 | 34.82 | 30.44 | 25.40 | 25.08 |
| | Useful aromatics | 8.25 | 5.22 | 5.70 | 7.16 | 10.80 | 10.56 |
| Yield of (ratio carbon atoms residue/number carbon atoms in reaction residue [number of moles of in reaction of moles of raw materials]) | | 4.59 | 10.50 | 7.30 | 13.76 | 7.53 | 12.91 |

From the results of Tables 2-1 to 3-2, the most favorable useful component yield (35% or greater) was obtained with the BEA zeolite inclusing $Rb^+$ and the MOR zeolite including $Rb^+$.

Further, with the BEA zeolite including $Rb^+$, the more favorable useful aromatic yield was achieved, and no residue was generated. Conversely, although a certain level of the useful aromatic yield was achieved with the FAU zeolite including $Rb^+$, a large amount of the residue was generated. It was assumed that a side reaction was likely to occur, which increased the amount of the residue, because the FAU zeolite had a large pore size and was likely to grow benzene rings.

(Comparative Example 7)

A gaseous product and a THF-soluble product were extracted in the same manner as in Example 1, except that the solid catalyst of Production Example 1 was not used.

(Example 6)

A gaseous product and a THF-soluble product were extracted in the same manner as in Example 1, except that the addition of 0.2 g of the solid catalyst of Production Example 1 to 0.3 g of polyethylene, 0.3 g of polypropylene, and 0.3 g of polystyrene was changed to addition of 0.0045 g of the solid catalyst of Production Example 1 to 0.3 g of polyethylene, 3 g of polypropylene, and 0.3 g of polystyrene.

(Example 7)

A gaseous product and a THF-soluble product were extracted in the same manner as in Example 1, except that the addition of 0.2 g of the solid catalyst of Production Example 1 to 0.3 g of polyethylene, 0.3 g of polypropylene, and 0.3 g of polystyrene was changed to addition of 0.018 g of the solid catalyst of Production Example 1 to 0.3 g of polyethylene, 3 g of polypropylene, and 0.3 g of polystyrene.

(Example 8)

A gaseous product and a THF-soluble product were extracted in the same manner as in Example 1, except that the addition of 0.2 g of the solid catalyst of Production Example 1 to 0.3 g of polyethylene, 0.3 g of polypropylene, and 0.3 g of polystyrene was changed to addition of 0.27 g of the solid catalyst of Production Example 1 to 0.3 g of polyethylene, 3 g of polypropylene, and 0.3 g of polystyrene.

(Example 9)

A gaseous product and a THF-soluble product were extracted in the same manner as in Example 1, except that the addition of 0.2 g of the solid catalyst of Production Example 1 to 0.3 g of polyethylene, 0.3 g of polypropylene, and 0.3 g of polystyrene was changed to addition of 0.45 g of the solid catalyst of Production Example 1 to 0.3 g of polyethylene, 3 g of polypropylene, and 0.3 g of polystyrene.

[Evaluation of plastic degradability]

In the same manner as the evaluation of the plastic degradability in Examples 1 to 5 and Comparative Examples 1 to 6, analysis samples of the gaseous products and the THF-soluble products recovered in Comparative Example 7 and Examples 6 to 9 were prepared, the prepared analysis samples were analyzed by the gas chromatography (GC) device equipped with the flame ionization detector under the above analysis conditions, and each component was quantified based on the ratio between the peak area of each component and the peak area of the internal standard substance. The results are presented in Table 4 below.

[Quantification of reaction residue]

A reaction residue yield of each of Comparative Example 7 and Examples 6 to 9 was calculated in the same manner as the quantification of the reaction residues of Examples 1 to 5 and Comparative Examples 1 to 6. The results are presented in Table 4 below.

In Table 4 below, the "Added amount [phr]" of the solid catalyst denotes parts by mass of the added solid catalyst when the total mass (0.9 g) of polyethylene, polypropylene, and polystyrene is determined as 100 parts by mass.

[Table 4]

| | | Comp. Ex.7 | Ex. 6 | Ex. 7 | Ex. 1 | Ex. 8 | Ex. 9 |
|---|---|---|---|---|---|---|---|
| Solid catalyst | Type | - | Rb$^+$-substituted BEA zeolite (Production Ex. 1) | Rb$^+$-substituted BEA zeolite (Production Ex. 1) | Rb$^+$-substituted BEA zeolite (Production Ex. 1) | Rb$^+$-substituted BEA zeolite (Production Ex. 1) | Rb$^+$-substituted BEA zeolite (Production Ex. 1) |
| | Added amount [phr] | 0.0 | 0.5 | 2.0 | 22.2 | 30.0 | 50.0 |
| Yield [%] (based on carbon atoms) | Total of useful components | 38.03 | 37.72 | 44.16 | 44.39 | 50.03 | 42.25 |
| | Useful aromatics | 3.18 | 12.31 | 8.66 | 16.08 | 21.24 | 14.93 |
| Yield of reaction residue (ratio [number of moles of carbon atoms in reaction residue/ number of moles of carbon atoms in raw materials]) | | 2.41 | 0.00 | 0.00 | 0.00 | 0.00 | 0.00 |

EP 4 759 791 A1

The specific embodiments, production examples, and examples of the present disclosure have been described above, but the present disclosure is not limited by the above embodiments, production examples, and examples. The embodiments may be carried out in various forms, and various combinations, omissions, substitutions, additions, changes, and the like can be made without departing from the gist of the invention. These embodiments and modifications of the embodiments are included in the scope and gist of the invention, and are included in the invention specified in the claims and the equivalent scope of the claims.

The present international application claims priority to Japanese Patent Application No. 2023-131008 before the Japan Patent Office on August 10, 2023, the entire contents of which are incorporated herein by reference.

## Claims

1.  A method for producing an olefin-containing composition, the method comprising:
    a step of decomposing plastic in the presence of a zeolite to produce an olefin-containing composition including an olefin with 2 to 5 carbon atoms,
    wherein the zeolite is at least one selected from the group consisting of a beta zeolite including $Rb^+$ and a mordenite zeolite including $Rb^+$.

2.  The method for producing the olefin-containing composition according to claim 1,
    wherein the plastic is polyolefin.

3.  The method for producing the olefin-containing composition according to claim 1,
    wherein the plastic includes at least one selected from the group consisting of polyethylene, polypropylene, and polystyrene.

4.  The method for producing the olefin-containing composition according to claim 1 or 2,
    wherein the decomposing of the plastic is performed at a temperature of 300°C or higher and 1,100°C or lower.

5.  The method for producing the olefin-containing composition according to claim 1 or 2,
    wherein a molar ratio $[SiO_2/Al_2O_3]$ of $SiO_2$ to $Al_2O_3$ of each of the beta zeolite including $Rb^+$ and the mordenite zeolite including $Rb^+$ is 10 or greater and 10,000 or less.

6.  The method for producing the olefin-containing composition according to claim 1 or 2,
    wherein the beta zeolite including $Rb^+$ has a BET specific surface area of 400 $m^2$/g or greater and 500 $m^2$/g or less, and
    the mordenite zeolite including $Rb^+$ has a BET specific surface area of 300 $m^2$/g or greater and 400 $m^2$/g or less.

7.  The method for producing the olefin-containing composition according to claim 1, further comprising:
    a mixing step of obtaining a liquid mixture in which at least one selected from the group consisting of a beta zeolite and a mordenite zeolite, and a liquid including a rubidium compound are mixed; and
    a calcination step of calcining the liquid mixture in the air to obtain at least one selected from the group consisting of the beta zeolite including $Rb^+$ and the mordenite zeolite including $Rb^+$.

8.  The method for producing the olefin-containing composition according to claim 7,
    wherein the mixing step satisfies $1 \leq yz/x \leq 5,000$, where x (g) is a mass of at least one selected from the group consisting of the beta zeolite including $Rb^+$ and the mordenite zeolite including $Rb^+$, y is a molar ratio $[SiO_2/Al_2O_3]$ of $SiO_2$ to $Al_2O_3$ of at least one selected from the group consisting of the beta zeolite including $Rb^+$ and the mordenite zeolite including $Rb^+$, and z (mmol) is a number of moles of the rubidium compound.

9.  The method for producing the olefin-containing composition according to claim 7,
    wherein the beta zeolite is a beta zeolite including $H^+$, and
    the mordenite zeolite is a mordenite zeolite including $H^+$.

10. A catalyst for producing an olefin-containing composition, comprising:
    at least one selected from the group consisting of a beta zeolite including $Rb^+$ and a mordenite zeolite including $Rb^+$,

wherein the catalyst is used for producing an olefin-containing composition that includes an olefin with 2 to 5 carbon atoms.

## INTERNATIONAL SEARCH REPORT

International application No.

**PCT/JP2024/028594**

### A. CLASSIFICATION OF SUBJECT MATTER

*C07C 4/22*(2006.01)i; *B01J 29/18*(2006.01)i; *B01J 29/70*(2006.01)i; *B01J 37/02*(2006.01)i; *B01J 37/08*(2006.01)i; *C07B 61/00*(2006.01)i; *C07C 11/02*(2006.01)i; *C08J 11/10*(2006.01)i; *C10G 1/10*(2006.01)i
FI:   C07C4/22; C10G1/10; C07C11/02; C07B61/00 300; B01J37/02 101Z; B01J29/70 M; B01J29/18 M; B01J37/08 ZAB; C08J11/10

According to International Patent Classification (IPC) or to both national classification and IPC

### B. FIELDS SEARCHED

Minimum documentation searched (classification system followed by classification symbols)

C07C4/22; B01J29/18; B01J29/70; B01J37/02; B01J37/08; C07B61/00; C07C11/02; C08J11/10; C10G1/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2022/039094 A1 (SUMITOMO CHEMICAL COMPANY, LIMITED) 24 February 2022 (2022-02-24)<br>claims, examples | 1-10 |
| A | WO 2021/166854 A1 (SUMITOMO CHEMICAL COMPANY, LIMITED) 26 August 2021 (2021-08-26)<br>claims, examples | 1-10 |
| A | JP 6-220458 A (HITACHI, LTD.) 09 August 1994 (1994-08-09)<br>table 2 | 1-10 |
| A | 上道芳夫　他, ポリオレフィンの接触分解による低級オレフィン化, ファインケミカル, 2017, vol. 46, no. 12, pp. 44-51, (UEMICHI, Yoshio et al., Fine chemical), non-official translation (Catalytic degradation of polyolefin into lower olefins)<br>table 1 | 1-10 |

✓ Further documents are listed in the continuation of Box C.      ✓ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 October 2024** | **29 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

EP 4 759 791 A1

**INTERNATIONAL SEARCH REPORT**

| International application No. |
|---|
| **PCT/JP2024/028594** |

**C.    DOCUMENTS CONSIDERED TO BE RELEVANT**

| Category* | Citation of document, with indication, where appropriate, of the relevant passages | Relevant to claim No. |
|---|---|---|
| A | 加賀慎之介　他, ポリエチレンの接触分解による低級オレフィン化, 石油学会　年会・秋季大会講演要旨集　第４７回石油・石油化学討論会（鳥取）, 2017, p. 190, (KAGA, Shinnosuke et al., Catalytic degradation of polyethylene into lower olefins, Proceeding of Annual/Fall Meetings of the Japan Petroleum Institute: The 47th Petroleum-Petrochemical Symposium of JPI (Tottori))<br>    table 1 | 1-10 |

Form PCT/ISA/210 (second sheet) (July 2022)

**INTERNATIONAL SEARCH REPORT**
Information on patent family members

International application No.

**PCT/JP2024/028594**

| Patent document cited in search report | | | Publication date (day/month/year) | Patent family member(s) | | | Publication date (day/month/year) |
|---|---|---|---|---|---|---|---|
| WO | 2022/039094 | A1 | 24 February 2022 | US | 2023/0357105 | A1 | |
| | | | | claims, examples | | | |
| | | | | EP | 4201524 | A1 | |
| | | | | CN | 115956006 | A | |
| | | | | KR | 10-2023-0054399 | A | |
| WO | 2021/166854 | A1 | 26 August 2021 | US | 2023/0113926 | A1 | |
| | | | | claims, examples | | | |
| | | | | EP | 4108649 | A1 | |
| | | | | CN | 115151519 | A | |
| | | | | KR | 10-2022-0143816 | A | |
| JP | 6-220458 | A | 09 August 1994 | (Family: none) | | | |

Form PCT/ISA/210 (patent family annex) (July 2022)

REFERENCES CITED IN THE DESCRIPTION

**Patent documents cited in the description**

- WO 2023064741 A
- JP 2023504126 A

- JP 2023131008 A

**Non-patent literature cited in the description**

- **WANG et al.** *International Journal of Metallurgical and Materials Engineering*, 2021, vol. 6 (4), 260-264

- Pore size distribution and porosity of solid materials by mercury porosimetry and gas adsorption-Part 3: Analysis of micropores by gas adsorption. *ISO 15901-3:2007*